# EUROPÄISCHE PATENTANMELDUNG

(11) **EP 1 262 777 A2**
(43) Veröffentlichungstag der Anmeldung: **04.12.2002**
(21) Anmeldenummer: 02010417.0
(22) Anmeldetag: 08.05.2002
(51) Int. Cl.: G01N 33/569, G01N 33/68, G01N 33/543

(54) **Nachweis von Erregern und durch sie induzierte zelluläre Stoffe in vivo**

(30) Priorität: 17.05.2001 DE 10125730
(71) Anmelder: A.I.D. Autoimmun Diagnostika GmbH, 72479 Strassberg (DE)
(72) Erfinder: Schöllhorn, Volkmar, Dr., 72479 Strassberg (DE)
(74) Vertreter: Patentanwälte Ruff, Wilhelm, Beier, Dauster & Partner

(57) **Zusammenfassung**

Die Erfindung betrifft ein Verfahren zum Nachweis von durch Erreger induzierten zellulär produzierten Stoffen oder von Erregern selbst, insbesondere zur Diagnose von subakuten spongiformen Enzephalopathien im lebenden Organismus.

## Beschreibung

Die Erfindung betrifft ein Verfahren zum Nachweis von durch Erreger induzierten zellulär produzierten Stoffen oder von Erregern selbst, insbesondere zur Diagnose von subakuten spongiformen Enzephalopathien im lebenden Organismus.

Bei den Enzephalopathien handelt es sich um eine Sammelbezeichnung für nicht entzündliche Erkrankungen oder Schädigungen des Gehirns. Bei den subakuten spongiformen Enzephalophatien (heutzutage häufig als transmissible (übertragbare) spongiforme Enzephalopathien bezeichnet), handelt es sich um eine Gruppe sporadischer, erblich oder übertragbarer Erkrankungen des ZNS, die durch schwammige (spongiforme) Degenerationen des Gehirns (ausgedehnter Nervenzellverlust, Wucherung der Neuroglia) und langsam progredienten, immer tödlichen Verlauf gekennzeichnet sind und den Prionkrankheiten zugerechnet werden.

Im Tierreich kommen diese Formen der Erkrankungen bei Schaf (scrapie), Rind (BSE) und anderen Säugetierarten vor. Beim Menschen wird die Creutzfeld-Jakob-Krankheit, Kuru, Gerstmann-Sträussler-Scheinker-Syndrom und die tödliche familiäre Schlaflosigkeit diesen Erkrankungen zugerechnet. BSE (bovine spongiform encephalopathie; sogenannter Rinderwahnsinn) ist eine bei Rindern vorkommende Form der subakuten spongiformen Enzephalophatien, die seit 1985 vor allem in Großbritannien epidemiologisch auftreten. Bis 1996 sind in Großbritannien mehr als 160.000 Fälle von BSE registriert geworden, und auch in anderen Ländern wie Frankreich, Schweiz, Deutschland ist BSE nachgewiesen geworden.

Ursache für BSE ist vermutlich die Verfütterung unzureichend sterilisierten Tiermehls, das aus Kadavern von scrapie-infizierten Schafen hergestellt wurde. Eine Übertragung auf den Menschen durch den Verzehr insbesondere von Rinderschlachtprodukten (vor allem Gehirn, Innereien) ist nicht auszuschließen, und insbesondere im Zusammenhang mit der "neuen" Form von Creutzfeld-Jakob-Krankheit wird die Übertragung von BSE auf den Menschen diskutiert.

Bei den Prionen (proteinaceous infectious particles; eigentlich Proinen) handelt es sich um von Viren, Viroiden und Plasmiden unterscheidbare infektiöse Eiweißpartikel (MG ca. 28.000), die eventuell kleinen Nukleotiden zusammen mit einem Polypeptid entsprechen, und von einem Wirtsgen (PrP-Gen) kodiert werden; Das primär apathogene Genprodukt PrP^{C} geht durch Konformationsänderung (eventuell durch Chaperone beschleunigt) in die infektiöse Isoform PrP^{Sc} über, die in akkumulierter Form als stab- oder fibrillenförmige Partikel (sogenannte Prionstäbe, scrapie-associated fibrils) im ZNS von Tieren und Menschen mit subakuten spongiformen Enzephalophatien nachgewiesen werden kann.

Erstaunlicherweise konnte bis jetzt kein Hinweis auf Antigene oder eine Immunantwort des Wirtes auf die infektiösen Partikel nachgewiesen werden, und die Prionen zeigen eine ungewöhnliche Resistenz gegenüber Proteasen, Nukleasen, Temperatur, UV- und Röntgenstrahlung sowie chemischen Einflüssen. Die oben beschriebenen Prionkrankheiten zeichnen sich vor allem dadurch aus, daß sie sporadisch oder familiär gehäuft auftreten und durch Gewebeinokulation bzw. Einweißinjektion übertragbar sind. Gemeinsame Merkmale der Prionenkrankheiten sind spezifische Mutationen des PrP-Gens bei familiärem Vorkommen, die lange Latenzzeit (meist mehrere Jahre), der unaufhaltsam progrediente, stets tödlich endende Verlauf und pathologisch-anatomisch das Fehlen klassischer Entzündungszeichen bei schwammiger Degeneration des Hirngewebes. Die Überwindung von Artenbarrieren durch Prionen ist innerhalb des Tierreiches erwiesen (z.B. zwischen Schaf und Rind), und eine Übertragbarkeit von Tieren auf den Menschen ist nicht auszuschließen und sehr wahrscheinlich.

Neben der Gefahr für die Volksgesundheit sind es vor allem die finanziellen Schäden, die durch BSE entstanden sind und sich mittlerweile zu Milliardenbeträgen summieren, die die Suche nach geeigneten Tests und Nachweisverfahren vorantreiben. Die Prionproteine kommen vor allem im Gehirn und Rückenmark vor, weil sich hier viele Synapsen der Nervenbahnen befinden, in denen sich die zellulären Prionenproteine anreichern. Auf diesem Tatbestand basieren zwei unterschiedliche Testprinzipien, die in Deutschland zum Nachweis von BSE etabliert sind: Einer spürt das Risikogewebe Hirn und Rückenmark, das Zentralnervengewebe (ZNS), in Wurstwaren auf. Der andere weist das veränderte Prionprotein PrP^{BSE} selbst nach. Von diesen Verfahren existieren einige Varianten (Enfer Scientific, Biorad Laboratories). Beiden Tests ist gemeinsam, daß sie mit spezifischen Antikörpern und der Western Plot-Technik arbeiten. Bei dem direkten Nachweis des BSE-Erregers wird Gewebe aus dem Hirn und dem Rückenmark des Rindes entnommen, dieses Gewebe homogenisiert und durch eine Protease verdaut, SDS-Gel elektrophoretisch aufgetrennt und mit Hilfe eines spezifischen monoklonalen Antikörpers und der Chemilumineszenztechnik nachgewiesen.

Da aber erst ca. 6 Monate vor Ausbruch der Erkrankung die Konzentration der PrP^{BSE} in dem Gewebe einen Wert erreicht, der nachgewiesen werden kann, und die Inkubationszeit beim Rind zwischen 24 Monaten und 6 Jahren, durchschnittlich bei 5 Jahren liegt, sowie in Deutschland 60 % der Rinder, die geschlachtet werden, nicht älter als 2 Jahre werden, ist der Hauptteil des Rindfleisches, das in Deutschland in die Nahrungskette gelangt, von Tieren abstämmig, die, auch wenn sie BSE hätten, mit diesem Testverfahren nicht erkannt werden könnten. Bei dem zweiten Nachweisverfahren werden nicht direkt Prionen, sondern ZNS-Gewebe nachgewiesen. So lassen sich insbesondere Wurstwaren daraufhin untersuchen, ob Hirn oder Rückenmark verarbeitet wurde, welches nicht mehr verwendet werden darf. Durch diese Methode läßt sich ZNS-Gewebe vom Schwein und Rind in der Wurst bis zu einem Gehalt von 0,25%, durch Filtrationskonzentrieren bis auf 0,01% nachweisen, jedoch ist dies kein direkter Nachweis der infektiösen Erreger, und eine Eindämmung der Krankheit kann nicht erfolgen, da erst nach Verarbeitung und Gewebeentnahme am Schlachtvieh, wie auch beim direkten Nachweisverfahren, der Nachweis erfolgen kann.

Erwünscht wäre ein Testverfahren für das lebende Tier, insbesondere indem im Blut des BSE-infizierten Tieres ein Markerprotein nachgewiesen werden könnte. Jedoch ist bis heute kein Produkt entwickelt worden, das für diesen BSE-Nachweis geeignet ist. Es ist noch nicht einmal bekannt, ob BSE-Erreger oder Folgeprodukte im Blut vorkommen. Dies liegt insbesondere daran, daß viele Fragen bezüglich der Prionenkrankheiten und insbesondere von BSE noch nicht aufgeklärt worden sind. So ist zum Beispiel noch nicht geklärt, wie genau der Übertragungsweg von BSE ist. Es wird vermutet, daß Prionen im Verdauungstrakt aufgenommen werden und von Immunzellen weitergeleitet werden. In den Lymphknoten scheinen sich diese dann anzureichern, bevor nach einer mehrjährigen Latenzzeit die Übertragung auf die Nervenzellen erfolgt. Da aber das Entfernen der Lymphknoten ein hohen präparativen Aufwand darstellt und am lebenden Tier nicht durchgeführt wird, scheidet der Nachweis von Prionen in Lymphknoten als Testverfahren aus. Ferner ist zu berücksichtigen, daß die Sensitivität üblicher Nachweisverfahren zu gering ist, um eine Infektion in einem frühen Stadium festzustellen. So ist es, wie oben bereits dargestellt, erst bei oder kurz vor Ausbruch der Erkrankung möglich, die akkumulierten "krankmachenden" Prionen zu detektieren, weil erst dann die Konzentration dieser hoch genug ist um innerhalb der Nachweisgrenze der Testvefahren zu liegen. Dieser Zeitpunkt ist aber für eine Überwachung der Tierbestände und eventuelle Eindämmung der Epidemie zu spät, da die infizierten Tiere schon in die Nahrungskette gelangt sind.

Ziel der Erfindung ist es daher, ein Verfahren zum Nachweis von durch Erreger induzierte zellulär produzierten Stoffe oder des Erregers selbst bereitzustellen, was spezifisch und sensitiv genug ist, bereits in einer sehr frühen Phase der Infektion am lebenden Organismus die entsprechende Infektion nachzuweisen.

Dieses Ziel wird gelöst durch das Verfahren wie es in Anspruch 1 definiert ist. Bevorzugte Ausführungsformen sind in den abhängigen Ansprüchen 2 bis 23 dargelegt. Der Wortlaut sämtlicher Ansprüche wird hiermit durch Bezugnahme zum Inhalt dieser Beschreibung gemacht.

Überraschenderweise wurde gefunden, daß Immunzellen, vor allem B-Zellen und follikuläre dendritische Zellen (FDC) für den Transport von Prionen in das neuronale Gewebe zuständig sind. Prionen, die für die subakute/transmissible spongiforme Enzephalopathie verantwortlich sind, sind in den B-Zellen, aber auch in den T-Zellen, des lymphatischen Gewebes nachgewiesen worden. Bisher konnten Sie aber nicht in Blutlymphozyten nachgewiesen werden. Mit Hilfe eines hochsensitiven Nachweisverfahrens, des sogenannten ELISA-Spot, konnte nun der Nachweis erbracht werden, das schon in Blutzellen Prionen gefunden werden können.

Gegenstand der Erfindung ist somit ein Verfahren zum Nachweis von durch Erreger induzierte zellulär produzierte Stoffe oder des Erregers selbst, insbesondere zur Diagnose von subakute spongiforme Enzephalophatien, wobei vom lebenden Organismus eukaryotische Zellen, insbesondere menschliche oder tierische Zellen, entnommen werden, und diese Zellen in Gegenwart von stoff- oder erregerspezifischen Fremdstoffen inkubiert werden, wobei die zellulär produzierten Stoffe oder der Erreger mit dem stoff- oder erregerspezifischen Fremdstoffen interagieren, und diese Interaktion durch eine Farbreaktion nach der ELISA-Spot-Methode (Elispot) nachgewiesen wird. Die Elispotmethode ist wesentlich empfindlicher als der ELISA-Test oder die FACS-Analyse. Der ELISA-Spot kann eine prionen-beladene Zelle aus über eine Millionen nicht infizierter Zellen herausfinden. Daher kann der Test sowohl für die Früherkennung von Erkrankungen, z.B. die Früherkennung von BSE-infizierten Rindern und das rechtzeitige Schlachten dieser, sowie auch des Monitorings, d.h. der Überwachung von Tierbeständen, benutzt werden. Ferner ist auch die Früherkennung und das Monitoring beim Menschen möglich. Unter geeigneten Farbreaktionen sind zum Beispiel die Lumineszenz- und Fluoreszenzfärbungen zu nennen.

Der Vorteil der Elispot-Methode besteht darin, daß zum Nachweis von infizierten Zellen nicht die Anzahl der reaktiven Zellen gemessen wird, um daraus Rückschlüsse auf den Grad der Infektion ziehen zu können, sondern vor allem wird die Qualität der Reaktion einzelner Zellen bestimmt. So können einzelne Zellen sehr unterschiedlich auf gleiche Reizstoffe reagieren, was sich in der Quantität der abzugebenden bzw. zu dedektierenden Stoffe und damit in der örtlichen Quantität der Farbreaktion jeder einzelnen Zelle ausdrückt. Daher ist es vorteilhaft, nicht nur die Anzahl der reaktiven Zellen zu ermitteln, sondern die Intensität der Gesamtreaktion zu bestimmen. Dieses erlaubt wesentlich weitergehende Schlüsse auf den Grad der Infektion einer Zelle bzw. höhere Sensitivitäten falls der Nachweis einer Infektion erbracht werden soll. So ist es möglich, mit Hilfe der Elispot-Methode und verschiedener Elispot-Variationen wesentlich zuverlässigere und aussagekräftigere Auswertungen durchzuführen.

Gemäß einer bevorzugten Ausführungsform der Erfindung handelt es sich bei den menschlichen oder tierischen Zellen um Blutzellen. Die Blutzellen können vor der Inkubation angereichert werden, um so die Sensitivität und Spezifität des Nachweises zu erhöhen. Bei den Blutzellen handelt es sich um Lymphozyten, insbesondere um B-Zellen, aber auch T-Zellen können für das erfindungsgemäße Nachweisverfahren benutzt werden.

Gemäß einer bevorzugten Ausführungsform handelt es sich bei den zellulär produzierten Stoffen oder Erregern um Prionen, Viren und/oder Bakterien bzw. um unter deren Kontrolle zellulär produzierte Stoffe, die durch eine Farbreaktion mit für diese Stoffe bzw. für diese Erreger spezifischen Fremdstoffen nachgewiesen werden. So existiert zum Beispiel ein monoklonaler Antikörper (6H4), der sowohl an Prionproteine bindet, die in einer veränderten (PrP^{Sc}) oder normalen Konformation (PrP^{C}) vorliegen. Bei den Prionen werden insbesondere die Prionen von der Erfindung umfaßt, die bei der Manifestation von Scrapie, BSE und/oder Creutzfeld-Jakob-Krankheit beteiligt sind, aber auch alle anderen Erreger, insbesondere Prionen, die mit Hilfe der Elispot-Methode im Blut nachgewiesen werden, kommen in Frage. Sofern es sich um ein Nachweisverfahren für Viren handelt, ist z.B. der Zytomegalie-Virus und/oder der Epstein-Barr-Virus erfindungsgemäß beansprucht.

In einer bevorzugten Ausführungsform handelt es sich bei den stoff- oder erregerspezifischen Fremdstoffen um Capture-Moleküle, insbesondere um Antikörper. Diese erregerspezifischen Fremdstoffe, insbesondere die Antikörper, werden dazu benutzt, um zum Beispiel auf einer Mikrotiterplatte gekoppelt zu werden, auf die dann später die zu untersuchenden Zellen draufgegeben werden, so daß die Zellen, die Erreger oder zellulär produzierte Stoffe haben, an örtlich benachbarte Antikörper binden und so einen Nachweis ermöglichen. Im allgemeinen können verschiedene zellulär produzierte Stoffe oder Erreger mit verschiedenen stoffoder erregerspezifischen Fremdstoffen spezifisch interagieren, wobei diese vorzugsweise spezifisch mit diesen binden.

In einer bevorzugten Ausführungsform führt die spezifische Interaktion zu mindestens zwei, vorzugsweise drei Farbreaktionen, wobei diese nach Art der Farbe getrennt erfaßt und ausgewertet werden können. Zum Beispiel ist es möglich mit Hilfe von verschiedenen Immunfluoreszenzfarbstoffen Prionen auf oder in den Zellen nachzuweisen, bzw. Prionen zu ermitteln, die ausgeschieden worden sind. Man kann ferner bei dieser Möglichkeit auch durch Nutzung von Fluorochromen mit unterschiedlichen Absorptionswellenlängen Mehrfachmarkierungen durchführen.

In einer besonders bevorzugten Ausführungsform der Erfindung werden Zellen in einem Untersuchungsgefäß, insbesondere in den Löchern von Mikrotiterplatten, fixiert und die zellulär produzierten Stoffe oder Erreger werden in oder auf den Zellen selbst, unter Nutzung geeigneter Nachweisverfahren wie zum Beispiel die oben beschriebenen Farbreaktionen, detektiert. Zum Fixieren sind beispielsweise Glutaraldehyd, Formamid, Aceton, Methanol, Ethanol und andere, dem Fachmann bekannte Fixierlösungen (Lösungsmittel), geeignet. Durch diese Fixierung ist es auch möglich, die einzelnen Zellpopulationen phänotypisch voneinander zu unterscheiden, zum Beispiel eine Typisierung von B- und/oder T-Zellen vorzunehmen. In dem nachfolgenden Nachweisverfahren kann dann geklärt werden, welche Zellen positiv sind, d.h. den Erreger oder die zellulär produzierten Stoffe tragen, enthalten bzw. abgeben. Alternativ dazu können die Zellen vor dem Nachweis lysiert werden. So können die dann abgegebenen zellulär produzierten Stoffe bzw. der Erreger selbst mit Hilfe eines Fremdstoffes weggefangen und nachgewiesen werden, insbesondere in unmittelbarer Umgebung der jeweiligen lysierten Zelle.

Gemäß einer besonders bevorzugten Ausführungsform werden die Zellen in einen stimulierten Zustand inkubiert. Dabei können die Zellen ex vivo stimuliert werden. Auch eine in vivo Stimulation ist möglich. Die Stimulation kann infolge einer natürlichen und/oder künstlichen Infektion erfolgen, so ist zum Beispiel eine Stimulierung durch eine Impfung möglich. Als Stimulanzien können zum Beispiel Mitogene und/oder Antigene genutzt werden. Mit Hilfe dieser Methoden ist eine Optimierung des geeigneten Zeitpunkts zum Beispiel der Blutentnahme möglich. So ist zum Beispiel beim gesundem Tier die "B-Zell-Traffic" sehr gering, d.h. die Zellen wandern nur zu einem sehr geringen Teil aus dem Lymphsystem ins Blut und wieder zurück. Zum Zeitpunkt einer Infektion ist dieser Traffic aber erhöht, so daß eine wesentlich höhere Chance zum Nachweis von beladenen (erregerbeladenen) Zellen besteht. Durch die vorhergehende Stimulierung bzw. die Ausnutzung einer natürlichen/künstlichen Infektion ist es möglich in einem sehr frühen Stadium der Erkrankung einen Nachweis zu erbringen. Macht man sich diese Methode zum Beispiel beim Nachweis von BSE zunutze, so ist es möglich infizierte, aber noch nicht erkrankte Tiere schnell aus der Herde auszusondern, ohne die anderen Tiere der Herde schlachten zu müssen. Anschließend kann man die Herde über Monate auf Neuinfektionen beobachten, d.h. die Herde überwachen und so nur die erkrankten Tiere, nicht aber die ganze Herde, notschlachten. Ferner kann diese Methode dafür genutzt werden, die Immunreaktion des Körpers anhand zahlreicher Immunparameter nach einer Prionen-Infektion zu messen, was zum Beispiel wichtig wäre für die Definition der Konzentration an Prionen, die zur Erkrankung führen, d.h., die Frage zu klären, wie viele Zellen im Blut müssen infiziert sein, um Symptome oder Infektionen hervorzurufen. Da zum Beispiel Prionen in der Regulation und Proliferation von Lymphozyten eine Rolle spielen, lassen sich als Stimulantien während der Proliferation von Lymphozyten Mitogene wie zum Beispiel Con-A nutzen. Durch die Stimulierung erfolgt eine Vermehrung der zellulär produzierten Stoffe oder des Erregers selbst. Weitere Stimulanzien sind dem Fachmann bekannt.

In einer bevorzugten Ausführungsform beträgt die Inkubationsdauer 30 Minuten bis 96 Stunden, vorzugsweise 4 bis 72 Stunden.

In einer weiteren bevorzugten Ausführungsform werden zur Durchführung des Nachweises mittels der ELISA-Spot-Methode Mikrotiterplatten verwendet. An den Lochboden der Mikrotiterplatten sind die verschiedenen stoff- oder erregerspezifischen Fremdstoffe fixiert, die dann mit den Zellen spezifisch interagieren, insbesondere diese binden. Bei bekannten Messungen nach der ELISA-Spot-Methode werden pro Loch der Mikrotitterplatte normalerweise eine Million Zellen eingesetzt. Im Blut sind 1 Million Zellen gewöhnlich in 1 ml Blut enthalten, so daß für eine 96-Loch-Platte schätzungsweise 100 ml Blut erforderlich ist. Es wurde erkannt, daß so große Zellmengen gar nicht erforderlich sind, weil ein Reader eines automatischen Auswertungsgerätes die Reaktion einzelner Zellen erfassen kann und eine wesentlich geringere Zellzahl ausreicht um statistisch gesicherte Ergebnisse erhalten zu können. Daher geht die Erfindung bei einer weiteren bevorzugten Ausführungsform den Weg, pro ELISA-Spot, insbesondere pro Loch auf der Mikrotiterplatte, 20.000 bis 200.000 Zellen zu inkubieren. Benutzt man jetzt nicht nur ein Loch, sondern 1 bis 40 Löcher, vorzugsweise 10 bis 30 Löcher für die Inkubation der Zellen, wie in einer weiteren bevorzugten Ausführungsform dargelegt, so kann man sich nach einer statistischen Überprüfung die Ergebnisse auf jeweils mehrere Millionen überprüfter Zellen beziehen. Mit der Elispot-Methode kann man pro Loch einer 96-Loch-Platte eine positive Zelle auf 200.000 Zellen erfaßen und entsprechend, beim Einsatz mehrerer Löcher, die Trefferzahl erhöhen. Bei dem erfindungsgemäßen Nachweisverfahren mit Hilfe der Elispot-Methode würde eine positive Zelle pro 1 Million ausreichen, d.h. eine auf 5 Löcher bei 200.000 Zellen pro Loch, um den Nachweis zu erbringen, d.h. eine infizierte B- oder T-Zelle aus 1 Million getesteter Zellen. Da anders als beim ELISA-Test bei der Elispot-Methode die Farbreagenz der Probe nicht löslich vorliegt, sondern sie sich über Stunden auf einem Punkt des Bodens anreichert, der durch eine Farbreaktion definiert werden kann, ist die hohe Sensitivität des Elispots gegeben. Bei der Detektion wird die hohe Empfindlichkeit des Elispots erhalten.

Im Vergleich zum Elispot ist zum Beispiel die Nachweisgrenze beim FACS 200 positive Zellen auf 200.000 eingesetzte Zellen, da beim FACS ca. 0,1% der Zellen positiv sein sollten, damit ein Nachweis erfolgen kann.

In einer weiteren bevorzugten Ausführungsform ist die Farbreagenz daher auch ohne vorhergehende Verdünnung auf die inkubierten Zellen zu geben. Wie bereits erwähnt, liegt bei der Elispot-Methode die Farbreagenz bzw. der gebundene Farbstoff nicht löslich vor. Als Farbreagenzien/Farbstoffe eignen sich alle Stoffe, die die Interaktion zwischen zellulär produzierten Stoffen oder Erreger und den stoff- oder erregerspezifischen Fremdstoffen nachweisen können, insbesondere ist dabei an Immunfluoreszenzfarbstoffe und Fluorochromfarbstoffe zu denken, die bevorzugt verwendet werden. Die Farbreagenzien/Farbstoffe binden dabei an, bzw. wandern in die zuvor fixierten Zellen selbst hinein. Dadurch entsteht ein von der Konzentration der Erreger bzw. der zellulär produzierten Stoffe abhängiger, örtlich fixierter Farbfleck, der dann mit üblichen Verfahren gemessen und ausgewertet wird.

Weitere Einzelheiten und Merkmale der Erfindung ergeben sich aus der nachfolgenden Beschreibung der Darstellung des erfindungsgemäßen Verfahrens und von bevorzugten Ausführungsformen in Verbindung mit den Unteransprüchen. Hierbei können die jeweiligen Merkmale für sich alleine oder zu mehreren in Kombination miteinander verwirklicht sein.

### Experiment 1:

Auf Anti-Prionen-beschichteten lmmobilon-Mikrotiterplatten werden diejenigen Zellen im Blut detektiert, die entweder ohne Stimulation, oder nach Stimulation mit Mitogenen, Prionen exprimieren oder in die Umgebung abgeben. Dabei werden auf die Mikrotiterplatte prionenspezifische Capture-Moleküle (Antikörper) gegeben und diese auf der Platte gekoppelt. Jetzt werden pro Loch ca. 100.000 bis 200.000 periphäre blutmononukleare Zellen (PBMCs) gegeben. Der Ansatz wird für 4 bis 72 Stunden inkubiert. Es werden gleichzeitig 10 bis 30 Löcher inkubiert. Prionen, die von einzelnen Zelle in gewissem Maße sezeniert bzw. auf diesen detektiert werden können, werden durch das Antikörpermolekül weggefangen und nach einer üblichen Farbreaktion nachgewiesen, z. B. mit Hilfe der Chemilumineszenztechnik.

### Experiment 2:

Dieser Ansatz basiert auf Experiment 1. Die Zellen werden hierbei nach einer Stimulierung lysiert, so daß nach dieser Zelllyse die lokal freigesetzten Prionen durch die Antikörper auf den Mikrotiterplatten gebunden und durch konjugierte Prionen-Antikörper nachgewiesen werden können.

### Experiment 3:

In diesem Ansatz werden Immobilon-Mikrotiterplatten (mit oder ohne Vorbeschichtung durch Anti-Prionen-Antikörper) mit Lymphozyten beschichtet und fixiert. Vorhandene Prionen werden fluoreszenzserologisch mit konjugierten Antikörpern nachgewiesen.

In allen drei Experimenten kann durch die Stimulierung mit entsprechenden Stimulantien (z.B. Con-A) die Proliferation verschiedener Lymphozyten angeregt werden, insbesondere auch der B-Zellen. Die stimulierten B-Zellen (aber auch andere Zellen) produzieren nun im größeren Maße Prionen. Diese Vermehrung der Prionen führt zum vermehrten Ausscheiden bzw. zu einer vermehrten Akkumulation der Prionen in der Zelle. Dadurch wird bei einer Farbreaktion eine erhöhte Sensitivität erreicht. Dabei ist zu beachten, daß normal produzierte Prionen, d.h. keine krank machenden Prionen, einem regelmäßigen Turnover unterliegen. Man kann daher davon ausgehen, daß sie nicht oder nur in einem sehr geringem Maße akkumuliert werden. Findet man also Zellen, die in einem hohen Maße Prionen akkumuliert haben, kann man davon ausgehen, daß diese enzymatisch nicht abgebaut werden bzw. nicht abgebaut wurden, was darauf schließen läßt, daß diese detektierten Prionen falsch gefaltet sind und dadurch enzymatisch nicht abbaubar sind. Diese sind dann die krankheitsverursachenden Prionen. Es ist daher nicht erforderlich zwischen richtig und falsch gefalteten Prionen zu unterscheiden.

Eine weitere Alternative um den Test sensitiver zu machen besteht darin, bei einer natürlichen oder künstlichen Infektion (beim Menschen oder beim Tier) oder anderen Stimulierungen das vermehrte Auftreten von B-Zellen im Blut durch den erhöhten Traffic zu nutzen. Dadurch ist auch die Wahrscheinlichkeit erhöht, Prionen beladene T-Zellen in periphärem Blut zu erfassen.

### Experiment 4:

Periphäres Blut oder bioptisches Material wird benutzt, um Viren aus Zellen mit Hilfe des Elispots zu detektieren. Antikörper gegen den Virus, im vorliegenden Fall gegen den Zytomegalie-Virus, werden auf den Mikrotiterplattenböden gekoppelt. Pro Loch werden hier 20.000 PBMCs für 4 bzw. 24 bis 48 Stunden inkubiert. Die Zellen werden mit Mitogen stimuliert, so daß sie zusätzlich große Mengen an Virus produzieren. Dieser Virus wird ausgeschleust und bindet an den Fangantikörpern, wo er mit Hilfe der üblichen Farbreaktion erfaßt werden kann. Auch hierbei läßt sich durch Testung von mehreren Löchern die Sensitivität des Assays drastisch steigern.

Der gleiche Ansatz kann auch bei anderen Viren wie HIV, EBV durchgeführt werden. Aus bioptischem Material (Leberbiopsie) kann man die Zellen ebenfalls nach enzymatischer Behandlung im Loch aufbringen und die Testmethode wie oben beschrieben durchführen.

## Patentansprüche

1. Verfahren zum Nachweis von durch Erreger induzierten zellulär produzierten Stoffen oder von Erregern selbst, insbesondere zur Diagnose von subakute spongiforme Enzephalopathien, **dadurch gekennzeichnet, daß** vom lebenden Organismus entnommene eukaryotische Zellen, insbesondere menschliche oder tierische Zellen, in Gegenwart von stoff- oder erregerspezifischen Fremdstoffen inkubiert werden, wobei die zellulär produzierten Stoffe oder Erreger mit den stoff- oder erregerspezifischen Fremdstoffen interagieren, und diese Interaktion durch eine Farbreaktion nach der ELISA-Spot-Methode nachgewiesen wird.

2. Verfahren nach Anspruch 1, **dadurch gekennzeichnet, daß** es sich bei den menschlichen oder tierischen Zellen um Blutzellen handelt, wobei insbesondere die Blutzellen vor der Inkubation angereichert werden.

3. Verfahren nach Anspruch 2, **dadurch gekennzeichnet, daß** es sich bei den Blutzellen um Lymphozyten, insbesondere um B-Zellen, handelt.

4. Verfahren nach einem der vorhergehenden Ansprüche, **dadurch gekennzeichnet, daß** es sich bei den zellulär produzierten Stoffen oder Erregern um Prionen, Viren und/oder Bakterien bzw. um unter deren Kontrolle zellulär produzierten Stoffen handelt und die Farbreaktionen mit für diese Stoffe bzw. für diese Erreger spezifischen Fremdstoffen durchgeführt werden, wobei es sich bei den Prionen insbesondere um solche handelt, die bei der Manifestation von Scrapie, BSE und/oder Creutzfeld-Jakob-Krankheit beteiligt sind, und es sich bei den Viren insbesondere um das Zytomegalie-Virus und/oder Epstein-Barr-Virus handelt.

5. Verfahren nach einem der vorhergehenden Ansprüche, **dadurch gekennzeichnet, daß** es sich bei den stoff- oder erregerspezifischen Fremdstoffen um Capture-Moleküle, insbesondere um Antikörper, handelt.

6. Verfahren nach einem der vorhergehenden Ansprüche, **dadurch gekennzeichnet, daß** verschiedene zellulär produzierten Stoffe oder Erreger mit verschiedenen stoff- oder erregerspezifischen Fremdstoffen spezifisch interagieren, vorzugsweise spezifisch mit diesen binden, wobei insbesondere die spezifischen Interaktionen zu mindestens zwei, vorzugsweise drei Farbreaktionen führen und diese nach Art der Farbe getrennt erfaßt und ausgewertet werden.

7. Verfahren nach einem der vorhergehenden Ansprüche, **dadurch gekennzeichnet, daß** die Zellen in einem Untersuchungsgefäß, insbesondere in den Löchern von Mikrotiterplatten, fixiert werden und die zellulär produzierten Stoffe oder Erreger in oder auf den Zellen detektiert werden.

8. Verfahren nach einem der Ansprüche 1 bis 6, **dadurch gekennzeichnet, daß** die Zellen vor dem Nachweis von zellulär produzierten Stoffe oder Erreger lysiert werden.

9. Verfahren nach einem der vorhergehenden Ansprüche, **dadurch gekennzeichnet, daß** die Zellen in einem stimulierten Zustand inkubiert werden, insbesondere daß die Zellen ex vivo stimuliert werden, und/oder die Zellen durch eine natürliche und/oder künstliche Infektion stimuliert sind, wobei insbesondere die Zellen durch eine Impfung stimuliert sind, und/oder die Zellen mit einem Mitogen und/oder Antigen stimuliert sind, und/oder durch die Stimulierung eine Vermehrung der zellulär produzierten Stoffe oder des Erregers selbst erfolgt.

10. Verfahren nach einem der vorhergehenden Ansprüche, **dadurch gekennzeichnet, daß** die Inkubationsdauer 30 Minuten bis 96 Stunden, vorzugsweise 4 bis 72 Stunden, beträgt.

11. Verfahren nach einem der vorhergehenden Ansprüche, **dadurch gekennzeichnet, daß** zur Durchführung des Nachweises mittels der ELISA-Spot-Methode Mikrotiterplatten verwendet werden.

12. Verfahren nach einem der vorhergehenden Ansprüche, **dadurch gekennzeichnet, daß** pro ELISA-Spot, insbesondere pro Loch auf der Mikrotiterplatte, 20.000 bis 200.000 Zellen inkubiert werden.

13. Verfahren nach Anspruch 11 oder 22, **dadurch gekennzeichnet, daß** die Zellen gleichzeitig auf 1 bis 40, vorzugsweise 10 bis 30, Löchern inkubiert werden.

14. Verfahren nach einem der vorhergehenden Ansprüche, **dadurch gekennzeichnet, daß** die Farbreagenz ohne vorhergehende Verdünnung auf die inkubierten Zellen gegeben wird.
